# EUROPEAN PATENT APPLICATION

(11) **EP 4 643 851 A1**
(43) Date of publication of application: **05.11.2025**
(21) Application number: 23912987.7
(22) Date of filing: 28.12.2023
(51) Int. Cl.: A61K 9/20, A61K 9/24, A61K 31/155, A61K 31/351

(54) **PHARMACEUTICAL COMPOSITION COMPRISING ENAVOGLIFLOZIN AND METFORMIN**

(30) Priority: 30.12.2022 KR 20220190502
(71) Applicant: Daewoong Pharmaceutical Co., Ltd., Hwaseong-si, Gyenoggi-do 18623 (KR)
(72) Inventor: PARK, Minhyung, Suwon-si Gyeonggi-do 16587 (KR); HWANG, On, Yongin-si Gyeonggi-do 16863 (KR); HA, Songyi, Yongin-si Gyeonggi-do 16863 (KR); KIM, Gyoungwon, Yongin-si Gyeonggi-do 16995 (KR); KIM, Hoe Sung, Yongin-si Gyeonggi-do 17028 (KR); CHO, Sangeun, Yongin-si Gyeonggi-do 16899 (KR); KIM, Gwan Young, Yongin-si Gyeonggi-do 16824 (KR)
(74) Representative: Plasseraud IP
(86) International application number: PCT/KR2023/021876
(87) International publication number: WO 2024/144313

(57) **Abstract**

The present invention relates to a pharmaceutical composition comprising enavogliflozin and metformin. The pharmaceutical composition, according to the present invention, makes it possible to implement an excellent preparation that provides the same level of medicinal efficacy as the combination therapy of a single tablet of metformin and a single tablet of enavogliflozin, despite a large content difference between metformin and enavogliflozin.

## Description

### [Technical Field]

The present invention relates to a pharmaceutical composition including enavogliflozin and metformin.

### [Background Art]

Metformin, which is an oral hypoglycemic agent of the biguanide class, is widely used as a first-line treatment for diabetes. Metformin has a blood gluscose improving effect, and exerts one of the known mechanisms of action through the activation of AMP-activated protein kinase (AMPK) in the liver, which leads to the inhibition of gluconeogenesis, enhancement of glucose uptake in cells, and suppression of metabolic syndrome.

Metformin has excellent glycated hemoglobin lowering effects, fewer side effects such as weight gain or hypoglycemia, and significantly reduces both diabetes-related mortality and overall mortality. However, metformin may cause gastrointestinal side effects such as diarrhea, abdominal discomfort, nausea, and vomiting, and requires caution when administered to patients with severe renal impairment or severe liver impairment.

Since cases in which blood glucose is not regulated only with metformin also frequently occur, drugs of the sulfonylurea class have been commonly prescribed as a second-line treatment for diabetes. However, insulin secretagogues such as sulfonylurea cause a decrease in pancreatic beta cells, which ultimately leads to a decrease in insulin secretion.

In addition, oral antidiabetic agents, such as an α-glucosidase inhibitor, a DPP4 inhibitor, and an SGLT-2 inhibitor, are also used for combination therapy with metformin.

A sodium-glucose cotransporter 2 (SGLT2) inhibitor is a new class of antihyperglycemic agent. An SGLT2 inhibitor increases glucose excretion through an insulin-independent mechanism by reducing glucose reabsorption in the proximal nephron. The safety and efficacy of SGLT2 inhibitors in the treatment of type 2 diabetes have been demonstrated in numerous studies.

Drugs that have been approved so far as SGLT2 inhibitors include dapagliflozin, empagliflozin, ipragliflozin, ertugliflozin, and enavogliflozin.

Enavogliflozin (compound name: (2S,3R,4R,5S,6R)-2-(7-chloro-6-(4-cyclopropylbenzyl)-2,3-dihydrobenzofuran-4-yl)-6-(hydroxymethyl)tetrahydro-2H-pyran-3,4,5-triol), a drug disclosed in U.S. Patent Application Publication No. 2015/0152075, was reported to exhibit an excellent blood glucose-lowering effect at a dose of only 0.3 mg, which is less than one-thirtieth of that of conventional SGLT2 inhibitors.

Metformin may be administered at a maximum of 2,000 mg per day, and has been developed in tablet forms with single-dose strengths of 500 mg, 750 mg, and 1,000 mg.

As formulations for combination therapy of metformin and an SGLT2 inhibitor, a composite composition of metformin and dapagliflozin, and a composite composition of metformin and empagliflozin have been developed. Based on 1,000 mg of metformin, the dapagliflozin content is approximately 10 mg, and the empagliflozin content is approximately 12.5 mg.

However, the single dose of enavogliflozin is 0.3 mg, which is only less than approximately one-thirtieth of that of a conventional SGLT2 inhibitor, and enavogliflozin is difficult to implement in a combination formulation unlike combination formulations containing metformin and a conventional SGLT2 inhibitor.

The content of metformin per tablet is 500 to 1,000 mg, but the content of enavogliflozin per tablet is 0.3 mg. The difference between the contents of the active ingredients is so large that it is very difficult to mix them uniformly when they are formulated into a single matrix tablet using a conventional formulation method. In addition, metformin is a drug that is highly soluble in water, and if formulated as a general tablet, it may cause excessive blood glucose reduction and gastrointestinal disorders due to rapid drug release, and therefore it is necessary to develop a sustained-release formulation to allow gradual dissolution. In this case, due to a high-viscosity swellable sustained-release agentused for sustained-release of metformin, there is a major problem that the release of enavogliflozin, which should be released immediately, may be delayed, and therefore it is difficult to expect metformin and enavogliflozin to be formulated as a single matrix tablet.

Although the case of AstraZeneca' Xigduo, which is a combination formulation of dapagliflozin and metformin, being formulated as a bilayer tablet can be considered, compared to the highest dose of Xigduo-10 mg of dapagliflozin and 1,000 mg of metformin-the dose difference between 0.3 mg of enavogliflozin and metformin 1,000 mg is too large, making it difficult to formulate these drugs as a bilayer tablet.

The total weight of the pharmaceutical composition of a single tablet of enavogliflozin disclosed in Korean Patent Application No. 10-2021-0130239 (Patent Document 1) is 75 mg. However, when the enavogliflozin part of the composite composition consists of the composition of the single tablet, the total amount of the enavogliflozin part is less than approximately 1/13 of the total amount of the metformin part including 1,000 mg of metformin. Because of this, it is difficult to meet the ratio conditions between the first and second drugs required in a bilayer tablet press, and therefore it is impossible to even attempt to prepare a bilayer tablet of a combination formulation with metformin based on the composition of the enavogliflozin single tablet.

### [Disclosure]

### [Technical Problem]

The present invention relates to a composite composition for combination therapy of metformin and enavogliflozin.

To provide the composite composition of metformin and enavogliflozin that can solve the above-described problems, a new formulation composition considering the difference in content between the two drugs is required, and at the same time, the composite composition must be able to achieve equivalent therapeutic efficacy to the combination therapy of a metformin single tablet and an enavogliflozin single tablet, which are control drugs.

### [Technical Solution]

As a result of various studies for formulating a composite composition of metformin and enavogliflozin, the present inventors confirmed that the above-described problems can be solved when the composition of a pharmaceutical composition is as follows.

Specifically, the present invention provides
a pharmaceutical composition in a single dosage form, which comprises a compartment including enavogliflozin or a pharmaceutically acceptable salt thereof and a compartment including metformin or a pharmaceutically acceptable salt thereof, the compartments being formulated in a separated form from each other,
wherein the enavogliflozin compartment is included at 10 to 20 parts by weight with respect to 100 parts by weight of the entire pharmaceutical composition, and
in the enavogliflozin compartment, enavogliflozin or a pharmaceutically acceptable salt thereof is included at less than 0.3 parts by weight with respect to a total of 100 parts by weight of the enavogliflozin compartment.

As described above, since the content of enavogliflozin in the formulation is very small, it is difficult to implement in a single matrix dosage form when formulating a composite composition with metformin. Therefore, it is preferable that the single-form pharmaceutical composition be formulated by separating the compartment including enavogliflozin or a pharmaceutically acceptable salt thereof (hereinafter, also referred to as 'enavogliflozin compartment' or 'enavogliflozin part') and the compartment including metformin or a pharmaceutically acceptable salt thereof (hereinafter, also referred to as 'metformin compartment' or 'metformin part').

Enavogliflozin used as an active ingredient in the present invention may be synthesized with reference to the known related documents. In the present invention, enavogliflozin may be crystalline or amorphous. For example, enavogliflozin may be crystalline form A, crystalline form B, crystalline form C, crystalline form D or crystalline form E of enavogliflozin, or amorphous enavogliflozin, which are reported to have the following X-ray diffraction spectra, according to Korean Unexamined Patent Application Publication No. 2017-0142904 or Korean Patent Application No. 2022-0123673.

Crystalline form A: the crystalline form with an X-ray diffraction (XRD) spectrum having peaks at 2[θ] values selected from 6.2° ± 0.2°, 7.2° ± 0.2°, 8.8° ± 0.2°, 17.6° ± 0.2°, 19.0° ± 0.2°, 22.5° ± 0.2°, and 25.1 ° ± 0.2°

Crystalline form B: the crystalline form with an X-ray diffraction (XRD) spectrum having peaks at 2[θ] values selected from 7.0° ± 0.2°, 14.9° ± 0.2°, 17.7° ± 0.2°, 18.8° ± 0.2°, 20.6° ± 0.2°, 21.8° ± 0.2°, and 23.5° ± 0.2°

Crystalline form C: the crystalline form with an X-ray diffraction (XRD) spectrum having peaks at 2[θ] values selected from 5.6° ± 0.2°, 7.3° ± 0.2°, 15.7° ± 0.2°, 17.2° ± 0.2°, 18.9° ± 0.2°, 21.2° ± 0.2°, and 21.9° ± 0.2°

Crystalline form D: the crystalline form with an X-ray diffraction (XRD) spectrum having peaks at 2[θ] values selected from 5.5° ± 0.2°, 7.2° ± 0.2°, 15.3° ± 0.2°, 17.2° ± 0.2°, 17.6° ± 0.2°, 18.9° ± 0.2°, and 21.1° ± 0.2°

Crystalline form E: the crystalline form with an X-ray diffraction (XRD) spectrum having peaks at 2[θ] values selected from 4.93°±0.2°, 6.12°±0.2°, 7.43°± 0.2°, 8.89°± 0.2°, 9.74°± 0.2°, 14.79°± 0.2°, 15.79°± 0.2°, 16.11°± 0.2°, 19.79°± 0.2°, and 22.83°±0.2°

Each of the crystalline forms A, B, C, D, and E can be specified by the X-ray diffraction spectra having four or more, for example, 4, 5, 6, 7, 8 or more, peaks at the presented 2[θ] values.

In one embodiment of the present invention, the average particle size of enavogliflozin may be 15 µm or less, and preferably 10 µm or less. When the average particle size of enavogliflozin is more than 15 µm, it may be difficult to achieve a desired dissolution rate.

In the present invention, the enavogliflozin compartment is included at 10 to 20 parts by weight with respect to 100 parts by weight of the entire pharmaceutical composition. When a combination formulation with separate compartments, such as a bilayer tablet, is normally prepared, the weight ratio of a first drug compartment and a second drug compartment ranges from 1:2 to 1:4. The above range is because when the total weight of the compartment of a drug with a smaller content is too small, the tablet may not be properly compressed.

When the enavogliflozin compartment is included at 10 to 20 parts by weight with respect to 100 parts by weight of the entire pharmaceutical composition, the metformin compartment is included at 90 to 80 parts by weight with respect to 100 parts by weight of the entire pharmaceutical composition. The weight ratio of the enavogliflozin compartment : the metformin compartment is 10 to 20 : 90 to 80, which is different from the composition of a conventional separate compartment formulation.

In addition, in the enavogliflozin compartment, enavogliflozin or a pharmaceutically acceptable salt thereof is included at less than 0.3 parts by weight with respect to a total of 100 parts by weight of the enavogliflozin compartment. The above content is different from that of the composition of an enavogliflozin single tablet (refer to Patent Document 1, containing 0.3 mg of enavogliflozin of the total content, 75 mg, of a tablet), including only enavogliflozin or a pharmaceutically acceptable salt thereof as an active ingredient.

More specifically, in the pharmaceutical composition according to the present invention, the enavogliflozin compartment includes enavogliflozin or a pharmaceutically acceptable salt thereof, an excipient, a disintegrant, and a glidant, and the metformin compartment includes metformin or a pharmaceutically acceptable salt thereof, a binder, a sustained-release agent, and a glidant.

In one embodiment, the enavogliflozin compartment includes an excipient selected from the group consisting of lactose monohydrate; mannitol; a mixture of microcrystalline cellulose and lactose monohydrate; and a mixture of microcrystalline cellulose, mannitol and pregelatinized starch.

The following example shows the change in dissolution rate of enavogliflozin according to the selection of an excipient. In the following example, lactose monohydrate alone; mannitol alone; a mixture of microcrystalline cellulose and lactose monohydrate; or a mixture of microcrystalline cellulose, mannitol and pregelatinized starch used as an excipient in the enavogliflozin compartment provides an equivalent dissolution rate for enavogliflozin when compared to the enavogliflozin single tablet.

The excipient is included at 80 to 85 parts by weight with respect to a total of 100 parts by weight of the enavogliflozin compartment, but the present invention is not limited thereto.

In one embodiment of the present invention, when the enavogliflozin compartment includes lactose monohydrate as an excipient, lactose monohydrate may be included at 40 to 100 parts by weight with respect to a total of 100 parts by weight of all excipients in the enavogliflozin compartment. When lactose monohydrate is used alone as an excipient, it accounts for a total of 100 parts by weight of the total excipient, and when lactose monohydrate is used along with microcrystalline cellulose, it may be used at 40 parts by weight or more and less than 100 parts by weight with respect to a total of 100 parts by weight of all excipients.

In one embodiment of the present invention, when the enavogliflozin compartment includes mannitol as an excipient, the mannitol may be included at 20 to 100 parts by weight with respect to a total of 100 parts by weight of all excipients in the enavogliflozin compartment. When mannitol is used alone as an excipient, it accounts for a total of 100 parts by weight of all excipients, and when mannitol is used along with microcrystalline cellulose and pregelatinized starch, it may be used at 20 parts by weight or more and less than 100 parts by weight with respect to a total of 100 parts by weight of all excipients.

In one embodiment of the present invention, when the enavogliflozin compartment includes microcrystalline cellulose as an excipient, an excessively large content of the microcrystalline cellulose reduces the dissolution of enavogliflozin and it is not preferable to use it alone as an excipient, and it is preferable to be use it in a mixture of microcrystalline cellulose and lactose monohydrate; or a mixture of microcrystalline cellulose, mannitol, and pregelatinized starch. The microcrystalline cellulose may be included at less than 65 parts by weight with respect to a total of 100 parts by weight of all excipients in the enavogliflozin compartment, but the present invention is not limited thereto.

In one embodiment of the present invention, when the enavogliflozin compartment includes pregelatinized starch as an excipient, the pregelatinized starch may be included at 5 to 40 parts by weight with respect to a total of 100 parts by weight of all excipients in the enavogliflozin compartment.

In another embodiment, when the enavogliflozin compartment includes microcrystalline cellulose, mannitol, and pregelatinized starch as excipients, mannitol may be included at 20 to 65 parts by weight with respect to a total of 100 parts by weight of all excipients in the enavogliflozin compartment.

In another embodiment, when the enavogliflozin compartment includes microcrystalline cellulose, mannitol, and pregelatinized starch as excipients, microcrystalline cellulose, mannitol, and pregelatinized starch may be included at a weight ratio of 1:1 to 1.5:0.15 to 1.5 in the enavogliflozin compartment. According to the present invention, it is elucidated that, when included at a high content in the enavogliflozin compartment, the microcrystalline cellulose tends to hinder the dissolution of enavogliflozin and reduce dissolution since it is not easily dissolved in water, compared to other components. Mannitol and pregelatinized starch may compensate for the disadvantages of microcrystalline cellulose and improve the dissolution rate of enavogliflozin.

In one embodiment of the present invention, the enavogliflozin compartment may include a disintegrant selected from the group consisting of croscarmellose sodium, sodium starch glycolate, crospovidone, and low-substituted hydroxypropyl cellulose. The disintegrant may be included at 5 to 20 parts by weight with respect to a total of 100 parts by weight of the enavogliflozin compartment. According to the following example, when the content of the disintegrant in the enavogliflozin compartment is 25 parts by weight or more, enavogliflozin does not achieve the dissolution rate of 80% or more within 10 minutes and the final dissolution rate of 85% or more, indicating an unfavorable dissolution profile in terms of bioavailability. This is because excessive use of the disintegrant can actually slow down the disintegration time and reduce the dissolution rate.

In one embodiment of the present invention, the enavogliflozin compartment includes one or more of light anhydrous silicic acid and talc as a glidant, and glidant may be included at 1.5 to 3 parts by weight with respect to a total of 100 parts by weight of the enavogliflozin compartment.

Meanwhile, in one embodiment of the present invention, the metformin compartment includes carboxymethylcellulose sodium, povidone, or a mixture thereof as a binder, and the binder may be included at 2 to 5 parts by weight with respect to a total of 100 parts by weight of the metformin compartment.

In another embodiment, the metformin compartment includes one or more of hydroxypropyl methylcellulose and oxidized polyethylene as a sustained-release agent, and the sustained-release agent may be included at 15 to 40 parts by weight with respect to a total of 100 parts by weight of the metformin compartment.

The hydroxypropyl methylcellulose preferably has an average viscosity of 100,000 mPa·s (75,000 ~ 140,000 mPa·s) to 200,000 mPa·s (150,000 ~ 280,000 mPa·s) in a 2% w/w solution, and the oxidized polyethylene preferably has an average molecular weight of 2,000,000 to 5,000,000. When the viscosity of the hydroxypropyl cellulose and the molecular weight of the oxidized polyethylene are low, incomplete formation of a polymer matrix for sustained release may affect the release rate of the drug.

In one embodiment of the present invention, the metformin compartment may include magnesium stearate as a glidant at 0.5 to 1 part by weight with respect to a total of 100 parts by weight of the metformin compartment.

As disclosed in Patent Document 1, due to the drug characteristics, the enavogliflozin compartment has Tₘₐₓ of 1 to 2 hours, and to achieve appropriate Cₘₐₓ and AUC, the drug is preferably formulated as an immediate-release type.

Preferably, in the pharmaceutical composition according to the present invention, the dissolution rate of the enavogliflozin or a pharmaceutically acceptable salt thereof in a pH 1.2 dissolution solution after 10 minutes may be 75% or more, and preferably 80% or more of the total content of the enavogliflozin or a pharmaceutically acceptable salt thereof.

Preferably, in the pharmaceutical composition according to the present invention, the dissolution rate of the enavogliflozin or a pharmaceutically acceptable salt thereof in a pH 1.2 dissolution solution after 45 minutes may be 80% or more, and preferably 85% or more of the total content of the enavogliflozin or a pharmaceutically acceptable salt thereof.

Since the dissolution rate of the active ingredient in the pharmaceutical composition affects the peak blood concentration (Cₘₐₓ) and area under the blood concentration-time curve (AUC) during drug administration, on the other hand, for the purpose of achieving appropriate Cₘₐₓ and AUC, it is important to adjust the dissolution rate of the pharmaceutical composition. Since enavogliflozin has a Tₘₐₓ of 1 to 2 hours, the drug absorption rate from above is considered important. The dissolution rate was measured under the condition of a pH 1.2 dissolution solution in accordance with the dissolution test method 2 (Paddle method). For specific conditions, the following experimental examples may be referenced.

When formulated as a conventional tablet, the metformin compartment can cause excessive hypoglycemia and gastrointestinal disorder due to rapid drug release, so it is preferably formulated as asustained-release type .

Preferably, in the pharmaceutical composition according to the present invention, the dissolution rate of the metformin or a pharmaceutically acceptable salt thereof in a pH 6.8 dissolution solution after 1 hour may be 15 to 35%, and preferably 20% or more of the total content of the metformin or a pharmaceutically acceptable salt thereof.

Preferably, in the pharmaceutical composition according to the present invention, the dissolution rate of the metformin or a pharmaceutically acceptable salt thereof in a pH 6.8 dissolution solution after 3 hours may be 40 to 60%, and preferably 45% or more of the total content of the metformin or a pharmaceutically acceptable salt thereof.

Preferably, in the pharmaceutical composition according to the present invention, the dissolution rate of the metformin or a pharmaceutically acceptable salt thereof in a pH 6.8 dissolution solution after 12 hours may be 80% or more, and preferably 85% or more of the total content of the metformin or a pharmaceutically acceptable salt thereof.

Metformin has a Tₘₐₓ of approximately 4 hours when administered before a meal and approximately 6 hours when administered after a meal; therefore, an intestinal drug absorption rate is regarded as crucial. The dissolution rate is measured under the condition of a dissolution solution of pH 6.8 in accordance with Korean Pharmacopoeia Dissolution Test Method 1 (Rotating Basket Method). Specific conditions are listed in the following experimental examples.

The pharmaceutical composition according to the present invention may further include pharmaceutically acceptable additives other than the above-described components. Examples of the additives include glidants and coloring agents.

The glidants include stearic acid, a stearate (e.g., magnesium stearate), light anhydrous silicic acid, talc, corn starch, carnauba wax, magnesium silicate, synthetic aluminum silicate, hydrogenated oil, white wax, titanium oxide, microcrystalline cellulose, macrogol 4000 and 6000, isopropyl myristate, calcium hydrogen phosphate, and a mixture thereof.

In one embodiment of the present invention, the enavogliflozin compartment includes a granulate in which pre-mixed granules including enavogliflozin or a pharmaceutically acceptable salt thereof are mixed with a post-mixed part.

The present inventors confirmed that, in the process of studying the formulation of enavogliflozin, it was confirmed that it is advantageous in terms of drug content uniformity and formulation uniformity to prepare granules and formulate them as a tablet.

The granules in the enavogliflozin compartment are prepared by mixing the pre-mixed granules with the post-mixed part.

The pre-mixed granules may include enavogliflozin or a pharmaceutically acceptable salt thereof, an excipient, and a glidant. In addition, the post-mixed part may include an excipient, a disintegrant, and a glidant.

The excipient, disintegrant, and glidant in the enavogliflozin compartment are the same as described above, and description will be omitted to avoid duplicate description.

In one embodiment of the present invention, each of the pre-mixed granules and post-mixed part in the enavogliflozin compartment may include one or more excipients.

In one embodiment of the present invention, in the enavogliflozin compartment, the pre-mixed granules may include microcrystalline cellulose, and the post-mixed part may include mannitol and pregelatinized starch.

The weight ratio of the excipient in the pre-mixed granules and the excipient in the post-mixed part in the enavogliflozin compartment may range from 1:1 to 1:4.

As the proportion of microcrystalline cellulose in the in the pre-mixed granules in the enavogliflozin compartment increases, the dissolution rate may decrease; therefore, it is confirmed that it is preferable to adjust a weight ratio within an appropriate range.

The granules in the enavogliflozin compartment in the pharmaceutical composition of the present invention may be dry granules, but the present invention is not limited thereto. Dry granulation enables the formation of granules with a suitable particle size distribution, which allows for excellent flowability and compression moldability during tableting, minimizing weight variation between individual tablets. The dry granulate plays an important role in preparing a bilayer tablet having a uniform content of enavogliflozin. In another embodiment, the granulate may be a wet granulate.

In one embodiment of the present invention, the metformin compartment includes a granulate in which pre-mixed granules including metformin or a pharmaceutically acceptable salt thereof are mixed with a post-mixed part.

The metformin compartment is preferably formed in a wet or dry granulate to avoid various problems during a formation process, such as tableting defects and coating defects due to a very high content and poor physical properties of metformin, which is the main component.

The granulate in the metformin compartment may be a wet granulate, but the present invention is not limited thereto. Alternatively, the granulate may be a dry granulate.

The granulate in the metformin compartment is prepared by mixing pre-mixed granules with a post-mixed part.

The pre-mixed granules may include metformin or a pharmaceutically acceptable salt thereof and a binder. In addition, the post-mixed part may include a sustained-release agent and a glidant.

The binder, sustained-release agent, and glidantin the metformin compartment are the same as described above, and thus description will be omitted to avoid duplicate description.

Meanwhile, in the present invention, the pharmaceutical composition may have a dosage form for oral administration, such as a tablet. In one embodiment of the present invention, the pharmaceutical composition may be formulated as a tablet. In a preferable embodiment, the pharmaceutical composition may be formulated as a bilayer tablet.

In one embodiment of the present invention, the pharmaceutical composition may include enavogliflozin at a dose of 0.1 to 0.5 mg, and preferably 0.15 to 0.3 mg.

In a preferable embodiment, the pharmaceutical composition may include metformin at a dose of 500 to 1,000 mg. Preferably, the pharmaceutical composition may include metformin at a dose of 500, 750, or 1,000 mg.

The pharmaceutical composition according to the present invention may be administered once a day, but the present invention is not limited thereto.

### [Advantageous Effects]

A pharmaceutical composition according to the present invention eables the realization of an excellent formulation that provides a level of efficacy equivalent to the combination therapy of a metformin single tablet and an enavogliflozin single tablet, despite the significant difference in content between metformin and enavogliflozin.

### [Description of Drawings]

FIG. 1 is a graph that compares the dissolution rates of enavogliflozin in formulations of Examples 1 to 4 and Comparative Examples 1 and 2 with that of a control drug under the condition of pH 1.2.
FIG. 2 is a graph that compares the dissolution rates of enavogliflozin in formulations of Examples 1, 5 and 6 and Comparative Example 3 with that of a control drug under the condition of pH 1.2.
FIG. 3 is a graph that compares the dissolution rates of enavogliflozin in formulations of Examples 1 and 7 to 10 and Comparative Example 4 with that of a control drug under the condition of pH 1.2.
FIG. 4 is a graph that compares the dissolution rates of metformin hydrochloride in formulations of Examples 1 and 11 to 17 with that of a control drug under the condition of pH 1.2.

### [Modes of the Invention]

Hereinafter, preferred examples are presented to help understand the present invention, but the following examples are only intended to illustrate the present invention and the scope of the present invention is not limited to the following examples. In addition, although the specification describes preferred methods and samples, similar or equivalent methods are also included within the scope of the present invention.

### [Examples]

### Example 1

### Step 1: Preparation of granules including enavogliflozin

Granules including enavogliflozin were prepared according to the composition of the enavogliflozin part of Table 1 below. The enavogliflozin was mixed with microcrystalline cellulose, light anhydrous silicic acid, and talc, and a plate-shaped compact was prepared using dry granules and then ground using a Comil to produce a dry granulate. Afterward, the dry granulate was mixed with mannitol, pregelatinized starch, croscarmellose sodium, talc, and a pigment blend to prepare enavogliflozin granules.

### Step 2: Preparation of granules including metformin hydrochloride

Granules including metformin hydrochloride were prepared according to the composition of a metformin part in Table 1 below. The metformin hydrochloride and carboxymethylcellulose sodium were mixed, combined using water as a binding solution, and dried. The resulting dried material was screened to prepare wet granules, and then hydroxypropyl methylcellulose (average viscosity: 100,000 mPa·s) and magnesium stearate were added and mixed with the wet granules to prepare metformin hydrochloride granules.

### Step 3: Preparation of combination tablet

A bilayer combination tablet, including 1,000 mg of metformin hydrochloride and 0.3 mg of enavogliflozin, was prepared by compressing them into a bilayer tablet which included metformin hydrochloride granules as a first layer and enavogliflozin granules as a second layer using a bilayer tablet press.

**[Table 1]**

| | Component | Example 1 |
|---|---|---|
| Enavogliflozin part | Enavogliflozin | 0.3 |
| | Microcrystalline cellulose | 55.0 |
| | Mannitol | 70.0 |
| | Pregelatinized starch | 40.0 |
| | Croscarmellose sodium | 30.0 |
| | Light anhydrous silicic acid | 2.0 |
| | Talc | 2.5 |
| | Pigment blend, orange | 0.2 |
| Metformin HCl part | Metformin HCl | 1,000.0 |
| | Carboxymethylcellulose sodium | 50.0 |
| | Hydroxypropyl methylcellulose | 300.0 |
| | Magnesium stearate | 10.0 |

### Comparative Examples 1 and 2

### Step 1: Preparation of granules including enavogliflozin

Granules including enavogliflozin were prepared according to the composition of an enavogliflozin part in Table 2 below.

Enavogliflozin was mixed with microcrystalline cellulose, light anhydrous silicic acid, and talc, and a plate-shaped compact was prepared using dry granules, and then ground using a Comil to produce a dry granulate. The dry granulate was mixed with croscarmellose sodium, talc, and a pigment blend to prepare enavogliflozin granules of Comparative Example 1.

The enavogliflozin was mixed with pregelatinized starch, light anhydrous silicic acid and talc, and a plate-shaped compact was prepared using dry granules and then ground using a Comil to produce a dry granulate. The dry granulate was mixed with pregelatinized starch, croscarmellose sodium, talc, and a pigment blend to prepare enavogliflozin granules of Comparative Example 2.

### Step 2: Preparation of granules including metformin hydrochloride

Granules including metformin hydrochloride were prepared according to the composition of a metformin part in Table 2 below. Metformin hydrochloride and carboxymethylcellulose sodium were mixed, combined using water as a binding solution, and dried. The resulting dried material was screened to prepare wet granules, and then hydroxypropyl methylcellulose (average viscosity: 100,000 mPa·s) and magnesium stearate were added and mixed with the wet granules to prepare metformin hydrochloride granules.

### Step 3: Preparation of combination tablet

A bilayer combination tablet, including 1,000 mg of metformin hydrochloride and 0.3 mg of enavogliflozin, was prepared by compressing them into a bilayer tablet which included metformin hydrochloride granules as a first layer and enavogliflozin granules as a second layer using a bilayer tablet press in the same manner as in Example 1.

**[Table 2]**

| | Component | Comparative Example 1 | Comparative Example 2 |
|---|---|---|---|
| Enavogliflozin part | Enavogliflozin | 0.3 | 0.3 |
| | Microcrystalline cellulose | 165.0 | |
| | Pregelatinized starch | | 165.0 |
| | Croscarmellose sodium | 30.0 | 30.0 |
| | Light anhydrous silicic acid | 2.0 | 2.0 |
| | Talc | 2.5 | 2.5 |
| | Pigment blend, orange | 0.2 | 0.2 |
| Metformin HCl part | Metformin HCl | 1,000.0 | 1,000.0 |
| | Carboxymethylcellulose sodium | 50.0 | 50.0 |
| | Hydroxypropyl methylcellulose | 300.0 | 300.0 |
| | Magnesium stearate | 10.0 | 10.0 |

### Examples 2 to 4

### Step 1: Preparation of granules including enavogliflozin

Granules including enavogliflozin were prepared according to the composition of an enavogliflozin part in Table 3 below.

Enavogliflozin was mixed with microcrystalline cellulose, lactose monohydrate, light anhydrous silicic acid, and talc, and a plate-shaped compact was prepared using dry granules, and then ground using a Comil to produce a dry granulate. The dry granulate was mixed with croscarmellose sodium, talc, and a pigment blend to prepare enavogliflozin granules of Example 2.

The enavogliflozin was mixed with lactose monohydrate, light anhydrous silicic acid, and talc, and a plate-shaped compact was prepared using dry granules and then ground using a Comil to produce a dry granulate. The dry granulate was mixed with pregelatinized starch, croscarmellose sodium, talc, and a pigment blend to prepare enavogliflozin granules of Example 3.

The enavogliflozin was mixed with mannitol, light anhydrous silicic acid, and talc, and a plate-shaped compact was prepared using dry granules and then ground using a Comil to produce a dry granulate. The dry granulate was mixed with mannitol, croscarmellose sodium, talc, and a pigment blend to prepare enavogliflozin granules of Example 3.

### Step 2: Preparation of granules including metformin hydrochloride

Granules including metformin hydrochloride were prepared according to the composition of a metformin part in Table 3 below. The metformin hydrochloride and carboxymethylcellulose sodium were mixed, combined using water as a binding solution, and dried. The resulting dried material was screened to prepare wet granules, and then hydroxypropyl methylcellulose (average viscosity: 100,000 mPa·s) and magnesium stearate were added and mixed with the wet granules to prepare metformin hydrochloride granules.

### Step 3: Preparation of combination tablet

A bilayer combination tablet, including 1,000 mg of metformin hydrochloride and 0.3 mg of enavogliflozin, was prepared by compressing them into a bilayer tablet which included metformin hydrochloride granules as a first layer and enavogliflozin granules as a second layer using a bilayer tablet press in the same manner as in Example 1.

**[Table 3]**

| | Component | Example 2 | Example 3 | Example 4 |
|---|---|---|---|---|
| Enavogliflozin part | Enavogliflozin | 0.3 | 0.3 | 0.3 |
| | Microcrystalline cellulose | 82.5 | | |
| | Lactose monohydrate | 82.5 | 165.0 | |
| | Mannitol | | | 165.0 |
| | Croscarmellose sodium | 30 | 30.0 | 30.0 |
| | Light anhydrous silicic acid | 2 | 2.0 | 2.0 |
| | Talc | 2.5 | 2.5 | 2.5 |
| | Pigment blend, orange | 0.2 | 0.2 | 0.2 |
| Metformin HCl part | Metformin HCl | 1,000.0 | 1,000.0 | 1,000.0 |
| | Carboxymethylcellulose sodium | 50.0 | 50.0 | 50.0 |
| | Hydroxypropyl methylcellulose | 300.0 | 300.0 | 300.0 |
| | Magnesium stearate | 10.0 | 10.0 | 10.0 |
| | | | | |

### Comparative Example 3

### Step 1: Preparation of granules including enavogliflozin

Granules including enavogliflozin were prepared according to the composition of an enavogliflozin part in Table 4 below.

Enavogliflozin was mixed with microcrystalline cellulose, light anhydrous silicic acid, and talc, and a plate-shaped compact was prepared using dry granules and then ground using a Comil to produce a dry granulate. The dry granulate was mixed with mannitol, pregelatinized starch, croscarmellose sodium, talc, and a pigment blend to prepare enavogliflozin granules of Comparative Example 3.

### Step 2: Preparation of granules including metformin hydrochloride

Granules including metformin hydrochloride were prepared according to the composition of a metformin part in Table 4 below.

Metformin hydrochloride was mixed with carboxymethylcellulose sodium, combined with water as a binding solution and then dried. The dried material was screened to prepare wet granules, and hydroxypropyl methylcellulose (average viscosity: 100,000 mPa·s) and magnesium stearate were added and mixed with the wet granules to prepare metformin hydrochloride granules.

### Step 3: Preparation of combination tablet

A bilayer combination tablet, including 1,000 mg of metformin hydrochloride and 0.3 mg of enavogliflozin, was prepared by compressing them into a bilayer tablet which included metformin hydrochloride granules as a first layer and enavogliflozin granules as a second layer using a bilayer tablet press in the same manner as in Example 1.

**[Table 4]**

| | Component | Comparative Example 3 |
|---|---|---|
| Enavogliflozin part | Enavogliflozin | 0.3 |
| | Microcrystalline cellulose | 55 |
| | Mannitol | 10 |
| | Pregelatinized starch | 100 |
| | Croscarmellose sodium | 30 |
| | Light anhydrous silicic acid | 2 |
| | Talc | 2.5 |
| | Pigment blend, orange | 0.2 |
| Metformin HCl part | Metformin HCl | 1,000.0 |
| | Carboxymethylcellulose sodium | 50.0 |
| | Hydroxypropyl methylcellulose | 300.0 |
| | Magnesium stearate | 10.0 |

### Examples 5 and 6

### Step 1: Preparation of granules including enavogliflozin

Granules including enavogliflozin were prepared according to the composition of an enavogliflozin part in Table 5 below.

Enavogliflozin was mixed with microcrystalline cellulose, light anhydrous silicic acid, and talc, and a plate-shaped compact was prepared using dry granules and then ground using a Comil to produce a dry granulate. The dry granulate was mixed with mannitol, pregelatinized starch, croscarmellose sodium, talc, and a pigment blend to prepare enavogliflozin granules of Examples 5 and 6.

### Step 2: Preparation of granules including metformin hydrochloride

Granules including metformin hydrochloride were prepared according to the composition of a metformin part in Table 5 below.

Metformin hydrochloride was mixed with carboxymethylcellulose sodium, combined with water as a binding solution and then dried. The dried material was screened to prepare wet granules, and hydroxypropyl methylcellulose (average viscosity: 100,000 mPa·s) and magnesium stearate were added and mixed with the wet granules to prepare metformin hydrochloride granules.

### Step 3: Preparation of combination tablet

A bilayer combination tablet, including 1,000 mg of metformin hydrochloride and 0.3 mg of enavogliflozin, was prepared by compressing them into a bilayer tablet which included metformin hydrochloride granules as a first layer and enavogliflozin granules as a second layer using a bilayer tablet press in the same manner as in Example 1.

**[Table 5]**

| | Component | Example 5 | Example 6 |
|---|---|---|---|
| Enavogliflozin part | Enavogliflozin | 0.3 | 0.3 |
| | Microcrystalline cellulose | 55.0 | 55.0 |
| | Mannitol | 100.0 | 40.0 |
| | Pregelatinized starch | 10.0 | 70.0 |
| | Croscarmellose sodium | 30.0 | 30.0 |
| | Light anhydrous silicic acid | 2.0 | 2.0 |
| | Talc | 2.5 | 2.5 |
| | Pigment blend, orange | 0.2 | 0.2 |
| Metformin HCl part | Metformin HCl | 1,000.0 | 1,000.0 |
| | Carboxymethylcellulose sodium | 50.0 | 50.0 |
| | Hydroxypropyl methylcellulose | 300.0 | 300.0 |
| | Magnesium stearate | 10.0 | 10.0 |
| | | | |

### Comparative Example 4

### Step 1: Preparation of granules including enavogliflozin

Granules including enavogliflozin were prepared according to the composition of an enavogliflozin part in Table 6 below.

Enavogliflozin was mixed with microcrystalline cellulose, light anhydrous silicic acid, and talc, and a plate-shaped compact was prepared using dry granules and then ground using a Comil to produce a dry granulate. The dry granulate was mixed with mannitol, pregelatinized starch, croscarmellose sodium, talc, and a pigment blend to prepare enavogliflozin granules of Comparative Example 4.

### Step 2: Preparation of granules including metformin hydrochloride

Granules including metformin hydrochloride were prepared according to the composition of a metformin part in Table 6 below.

Metformin hydrochloride was mixed with carboxymethylcellulose sodium, combined with water as a binding solution and then dried. The dried material was screened to prepare wet granules, and hydroxypropyl methylcellulose (average viscosity: 100,000 mPa·s) and magnesium stearate were added and mixed with the wet granules to prepare metformin hydrochloride granules.

### Step 3: Preparation of combination tablet

A bilayer combination tablet, including 1,000 mg of metformin hydrochloride and 0.3 mg of enavogliflozin, was prepared by compressing them into a bilayer tablet which included metformin hydrochloride granules as a first layer and enavogliflozin granules as a second layer using a bilayer tablet press in the same manner as in Example 1.

**[Table 6]**

| | Component | Comparative Example 4 |
|---|---|---|
| Enavogliflozin part | Enavogliflozin | 0.3 |
| | Microcrystalline cellulose | 55 |
| | Mannitol | 70 |
| | Pregelatinized starch | 40 |
| | Croscarmellose sodium | 50 |
| | Light anhydrous silicic acid | 2.0 |
| | Talc | 2.5 |
| | Pigment blend, orange | 0.2 |
| Metformin HCl part | Metformin HCl | 1,000.0 |
| | Carboxymethylcellulose sodium | 50.0 |
| | Hydroxypropyl methylcellulose | 300.0 |
| | Magnesium stearate | 10.0 |

### Examples 7 to 10

### Step 1: Preparation of granules including enavogliflozin

Granules including enavogliflozin were prepared according to the composition of an enavogliflozin part in Table 7 below.

Enavogliflozin was mixed with microcrystalline cellulose, light anhydrous silicic acid, and talc, and a plate-shaped compact was prepared using dry granules and then ground using a Comil to produce a dry granulate. The dry granulate was mixed with mannitol, pregelatinized starch, croscarmellose sodium, talc, and a pigment blend to prepare enavogliflozin granules of Example 7.

Enavogliflozin granules of Example 8 were prepared by mixing the dry granulate with mannitol, pregelatinized starch, sodium starch glycolate, talc, and a pigment blend.

Enavogliflozin granules of Example 9 were prepared by mixing the dry granulate with mannitol, pregelatinized starch, crospovidone, talc, and a pigment blend.

Enavogliflozin granules of Example 10 were prepared by mixing the dry granulate with mannitol, pregelatinized starch, and low-substituted hydroxypropyl cellulose.

### Step 2: Preparation of granules including metformin hydrochloride

Granules including metformin hydrochloride were prepared according to the composition of a metformin part in Table 7 below.

Metformin hydrochloride was mixed with carboxymethylcellulose sodium, combined with water as a binding solution and then dried. The dried material was screened to prepare wet granules, and hydroxypropyl methylcellulose (average viscosity: 100,000 mPa·s) and magnesium stearate were added and mixed with the wet granules to prepare metformin hydrochloride granules.

### Step 3: Preparation of combination tablet

A bilayer combination tablet, including 1,000 mg of metformin hydrochloride and 0.3 mg of enavogliflozin, was prepared by compressing them into a bilayer tablet which included metformin hydrochloride granules as a first layer and enavogliflozin granules as a second layer using a bilayer tablet press in the same manner as in Example 1.

**[Table 7]**

| | Component | Example 7 | Example 8 | Example 9 | Example 10 |
|---|---|---|---|---|---|
| Enavogliflozin part | Enavogliflozin | 0.3 | 0.3 | 0.3 | 0.3 |
| | Microcrystalline cellulose | 55.0 | 55.0 | 55.0 | 55.0 |
| | Mannitol | 70.0 | 70.0 | 70.0 | 70.0 |
| | Pregelatinized starch | 40.0 | 40.0 | 40.0 | 40.0 |
| | Croscarmellose sodium | 10.0 | - | - | - |
| | Sodium starch glycolate | - | 30.0 | - | - |
| | Crospovidone | - | - | 30.0 | - |
| | Low-substituted hydroxypropyl cellulose | - | - | - | 30.0 |
| | Light anhydrous silicic acid | 2.0 | 2.0 | 2.0 | 2.0 |
| | Talc | 2.5 | 2.5 | 2.5 | 2.5 |
| | Pigment blend, orange | 0.2 | 0.2 | 0.2 | 0.2 |
| Metformin HCl part | Metformin HCl | 1,000.0 | 1,000.0 | 1,000.0 | 1,000.0 |
| | Carboxymethylcellulose sodium | 50.0 | 50.0 | 50.0 | 50.0 |
| | Hydroxypropyl methylcellulose | 300.0 | 300.0 | 300.0 | 300.0 |
| | Magnesium stearate | 10.0 | 10.0 | 10.0 | 10.0 |

### Examples 11 to 14

### Step 1: Preparation of granules including enavogliflozin

Granules including enavogliflozin were prepared according to the composition of an enavogliflozin part in Table 8 below.

Enavogliflozin was mixed with microcrystalline cellulose, light anhydrous silicic acid, and talc, and a plate-shaped compact was prepared using dry granules and then ground using a Comil to produce a dry granulate. The dry granulate was mixed with mannitol, pregelatinized starch, croscarmellose sodium, talc, and a pigment blend to prepare enavogliflozin granules.

### Step 2: Preparation of granules including metformin hydrochloride

Granules including metformin hydrochloride were prepared according to the composition of a metformin part in Table 8 below.

Metformin hydrochloride was mixed with povidone, combined with water as a binding solution and then dried. The dried material was screened to prepare wet granules, and hydroxypropyl methylcellulose (average viscosity: 100,000 mPa·s) and magnesium stearate were added and mixed with the wet granules to prepare metformin hydrochloride granules of Example 11.

Metformin hydrochloride granules of Example 12 were prepared by adding hydroxypropyl methylcellulose (average viscosity: 200,000 mPa·s) and magnesium stearate to the wet granulate and mixing them.

Metformin hydrochloride granules of Example 13 were prepared by adding oxidized polyethylene (average molecular weight: 2,000,000) and magnesium stearate to the wet granulate and mixing them.

Metformin hydrochloride granules of Example 14 were prepared by adding oxidized polyethylene (average molecular weight: 5,000,000) and magnesium stearate to the wet granulate and mixing them.

### Step 3: Preparation of combination tablet

A bilayer combination tablet, including 1,000 mg of metformin hydrochloride and 0.3 mg of enavogliflozin, was prepared by compressing them into a bilayer tablet which included metformin hydrochloride granules as a first layer and enavogliflozin granules as a second layer using a bilayer tablet press in the same manner as in Example 1.

**[Table 8]**

| | Component | Example 11 | Example 12 | Example 13 | Example 14 |
|---|---|---|---|---|---|
| Enavogliflozin part | Enavogliflozin | 0.3 | 0.3 | 0.3 | 0.3 |
| | Microcrystalline cellulose | 55.0 | 55.0 | 55.0 | 55.0 |
| | Mannitol | 70.0 | 70.0 | 70.0 | 70.0 |
| | Pregelatinized starch | 40.0 | 40.0 | 40.0 | 40.0 |
| | Croscarmellose sodium | 30.0 | 30.0 | 30.0 | 30.0 |
| | Light anhydrous silicic acid | 2.0 | 2.0 | 2.0 | 2.0 |
| | Talc | 2.5 | 2.5 | 2.5 | 2.5 |
| | Pigment blend, orange | 0.2 | 0.2 | 0.2 | 0.2 |
| Metformin HCl part | Metformin HCl | 1,000.0 | 1,000.0 | 1,000.0 | 1,000.0 |
| | Povidone | 50.0 | 50.0 | 50.0 | 50.0 |
| | Hydroxypropyl methylcellulose (average viscosity: 100,000 mPa·s) | 300.0 | - | - | - |
| | Hydroxypropyl methylcellulose (average viscosity: 200,000 mPa·s) | - | 300.0 | - | - |
| | Oxidized polyethylene (average molecular weight: 2,000,000) | - | - | 300.0 | - |
| | Oxidized polyethylene (average molecular weight: 5,000,000) | - | - | - | 300.0 |
| | Magnesium stearate | 10.0 | 10.0 | 10.0 | 10.0 |

### Examples 15 to 17

### Step 1: Preparation of granules including enavogliflozin

Granules including enavogliflozin were prepared according to the composition of an enavogliflozin part in Table 9 below.

Enavogliflozin was mixed with microcrystalline cellulose, light anhydrous silicic acid, and talc, and a plate-shaped compact was prepared using dry granules and then ground using a Comil to produce a dry granulate. The dry granulate was mixed with mannitol, pregelatinized starch, croscarmellose sodium, talc, and a pigment blend to prepare enavogliflozin granules.

### Step 2: Preparation of granules including metformin hydrochloride

Granules including metformin hydrochloride were prepared according to the composition of a metformin part in Table 9 below. Metformin hydrochloride was mixed with carboxymethylcellulose sodium, combined with water as a binding solution and then dried. The dried material was screened to prepare wet granules, and hydroxypropyl methylcellulose (average viscosity: 200,000 mPa·s) and magnesium stearate were added and mixed with the wet granules to prepare metformin hydrochloride granules of Example 15.

Metformin hydrochloride granules of Example 16 were prepared by adding oxidized polyethylene (average molecular weight: 2,000,000) and magnesium stearate to the wet granulate and mixing them.

Metformin hydrochloride granules of Example 17 were prepared by adding oxidized polyethylene (average molecular weight: 5,000,000) and magnesium stearate to the wet granulate and mixing them.

### Step 3: Preparation of combination tablet

A bilayer combination tablet, including 1,000 mg of metformin hydrochloride and 0.3 mg of enavogliflozin, was prepared by compressing them into a bilayer tablet which included metformin hydrochloride granules as a first layer and enavogliflozin granules as a second layer using a bilayer tablet press in the same manner as in Example 1.

**[Table 9]**

| | Component | Example 15 | Example 16 | Example 17 |
|---|---|---|---|---|
| Enavogliflozin part | Enavogliflozin | 0.3 | 0.3 | 0.3 |
| | Microcrystalline cellulose | 55.0 | 55.0 | 55.0 |
| | Mannitol | 70.0 | 70.0 | 70.0 |
| | Pregelatinized starch | 40.0 | 40.0 | 40.0 |
| | Croscarmellose sodium | 30.0 | 30.0 | 30.0 |
| | Light anhydrous silicic acid | 2.0 | 2.0 | 2.0 |
| | Talc | 2.5 | 2.5 | 2.5 |
| | Pigment blend, orange | 0.2 | 0.2 | 0.2 |
| Metformin HCl part | Metformin HCl | 1,000.0 | 1,000.0 | 1,000.0 |
| | Carboxymethylcellulose sodium | 50.0 | 50.0 | 50.0 |
| | Hydroxypropyl methylcellulose (average viscosity: 200,000 mPa·s) | 300.0 | - | - |
| | Oxidized polyethylene (average molecular weight: 2,000,000) | - | 300.0 | - |
| | Oxidized polyethylene (average molecular weight: 5,000,000) | - | - | 300.0 |
| | Magnesium stearate | 10.0 | 10.0 | 10.0 |

### Experimental Example 1: Hardness test

A hardness test is a test that physically measures the degree of hardness of an oral dosage form (units: kP). The test was performed by placing a tablet on a hardness measuring device and measuring the hardness, and for testing, tablets prepared in the examples and comparative examples were used. The main compression pressure for each tablet was set at 25 to 28 kN, and the result of measuring the hardness of the tablets is shown in Table 10 below.

**[Table 10]**

| Hardness (kP) | | | | |
|---|---|---|---|---|
| Example 1 | Comparative Example 1 | Comparative Example 2 | Comparative Example 3 | Comparative Example 4 |
| 22.4 | 23.3 | 20.2 | 20.2 | 19.6 |
| Example 2 | Example 3 | Example 4 | Example 5 | Example 6 |
| 22.0 | 17.7 | 17.2 | 21.7 | 19.4 |
| Example 7 | Example 8 | Example 9 | Example 10 | Example 11 |
| 19.8 | 18.5 | 21.2 | 21.4 | 38.0 |
| Example 12 | Example 13 | Example 14 | Example 15 | Example 16 |
| 42.6 | 36.0 | 23.3 | 28.0 | 31.0 |
| Example 17 | | | | |
| 29.9 | | | | |

In the results of the hardness test, all prepared tablets showed a hardness of 15 kP or more, confirming that there were no problems in preparing the tablets.

### Experimental Example 2: Disintegration test

The disintegration test was performed on the enavogliflozin layers of Comparative Examples 1 to 4 and Examples 1 to 10 in accordance with the disintegration test in the General Test Methods of the Korean Pharmacopoeia. The results are shown in Table 11 below.

**[Table 11]**

| Disintegration time | | | | |
|---|---|---|---|---|
| Example 1 | Comparative Example 1 | Comparative Example 2 | Comparative Example 3 | Comparative Example 4 |
| 55 sec | 1 min 48 sec | 3 min 3 sec | 2 min 12 sec | 1 min 50 sec |
| Example 2 | Example 3 | Example 4 | Example 5 | Example 6 |
| 1 min | 1 min 40 sec | 1 min 23 sec | 55 sec | 1 min 28 sec |
| Example 7 | Example 8 | Example 9 | Example 10 | |
| 45 sec | 55 sec | 40 sec | 47 sec | |

The disintegration test is a test method to determine whether a tablet, a capsule, a granule, a pill, and a suppository disintegrate (disappear or disperse to a specified particle state) in a test solution under predetermined conditions within a specified time, which allows the prediction of the degree of dissolution in a solvent to some extent, but does not confirm whether the active ingredient in the formulation is completely dissolved. In the results of the disintegration test, it was confirmed that the comparative examples disintegrated relatively slower than the examples.

### Experimental Example 3: Stability evaluation

After the dosage form of Example 1 was stored in a sealed High-Density Polyethylene (HDPE) bottle under long-term test conditions (25 °C, 60% RH), enavogliflozin and metformin contents and the amounts of related substances were measured by a liquid chromatography analysis method at 3, 6, 9, and 12 months. After the dosage form of Example 1 was stored under accelerated test conditions (40 °C, 75% RH), enavogliflozin and metformin contents and the amounts of related substances were measured by a liquid chromatography analysis method at 1, 3, and 6 months. The results are shown in Tables 12 and 13 below.

**[Table 12]**

| Long-term test results (units: %) | | | | | | | |
|---|---|---|---|---|---|---|---|
| | | | Initial | 3 months | 6 months | 9 months | 12 months |
| Content | Enavogliflozin | | 99.9 | 10.5 | 102.5 | 99.6 | 102.1 |
| | Metformin | | 98.3 | 98.9 | 98.7 | 100.0 | 97.5 |
| Related substance | Enavogliflozin | Other related substances | 0.04 | 0.18 | 0.11 | 0.10 | 0.16 |
| | | Total related substances | 0.13 | 0.23 | 0.23 | 0.22 | 0.26 |
| | Metformin | Cyanoguanidine | N.D | N.D | N.D | N.D | N.D |
| | | Total related substances | N.D | N.D | N.D | N.D | N.D |
| NDMA (Standard: 48 ppb or less) | | | N/A | N.D | 0.83 ppb | 6.29 ppb | 5.15 ppb |

**[Table 13]**

| Accelerated test results (units: %) | | | | | | |
|---|---|---|---|---|---|---|
| | | | Initial | 1 month | 3 months | 6 months |
| Content | Enavogliflozin | | 99.9 | 100.7 | 99.9 | 101.3 |
| | Metformin | | 98.3 | 100.1 | 99.4 | 98.4 |
| Related substance | Enavogliflozin | Other related substances | 0.04 | 0.05 | 0.17 | 0.21 |
| | | Total related substances | 0.13 | 0.12 | 0.25 | 0.51 |
| | Metformin | Cyanoguanidine | N.D | N.D | N.D | N.D |
| | | Total related substances | N.D | N.D | N.D | N.D |
| NDMA (Standard: 48 ppb or less) | | | N/A | N/A | 10.57 ppb | 10.10 ppb |

As shown in Tables 12 and 13, through the stability tests using the contents and related substances of Example 1, stability was confirmed for 12 months under the long-term and accelerated test conditions. In addition, it was confirmed that the amount of N-nitrosodimethylamine (NDMA), which is a suspected carcinogen, was less than the maximum allowable daily dose (48 nanograms, 0.048 ppb), which is the provisional management standard, when taken at up to 2000 mg per day.

### Experimental Example 4: Dissolution test

The dissolution test was performed on enavogliflozin and metformin prepared in Comparative Examples 1 to 4 and Examples 1 to 17 under the same conditions as shown in Tables 14 and 15 below in accordance with the dissolution test method of the Korean Pharmacopoeia. To compare dissolution rates with that of the combination tablet of the present invention, the test was performed under the same conditions as the combination tablet of the present invention, using Daewoong Pharmaceutical's 'Envlo Tablet' (a single-agent formulation of enavogliflozin) and Merck's 'Glucophage XR, Extended-release tablet, 1,000 mg' (a single-agent formulation of metformin).

**[Table 14]**

| Dissolution test conditions for enavogliflozin | |
|---|---|
| Item | Condition |
| Dissolution test device | Agilent 708-DS |
| Dissolution test method | Method 2 (Paddle method) |
| Dissolution solution | pH 1.2 |
| Temperature of dissolution solution | 37.0 °C |
| Amount of dissolution solution | 500 mL |
| Rotating speed | 50 rpm |
| Analysis method | - Detector: UV spectrophotometer (measurement wavelength: 218 nm) |
| | - Mobile phase: Phosphate hydrogen buffer : Acetonitrile = 55 : 45 (v/v) |

**[Table 15]**

| Dissolution test conditions for metformin | |
|---|---|
| Item | Condition |
| Dissolution test device | Agilent 708-DS |
| Dissolution test method | Method 1 (Rotating Basket Method) |
| Dissolution solution | pH 6.8 |
| Temperature of dissolution solution | 37.0 °C |
| Amount of dissolution solution | 900 mL |
| Rotating speed | 100 rpm |
| Analysis method | - Detector: UV spectrophotometer (measurement wavelength: 218 nm) |
| | - Mobile phase: Phosphate hydrogen buffer : Acetonitrile = 85 : 15 (v/v) |

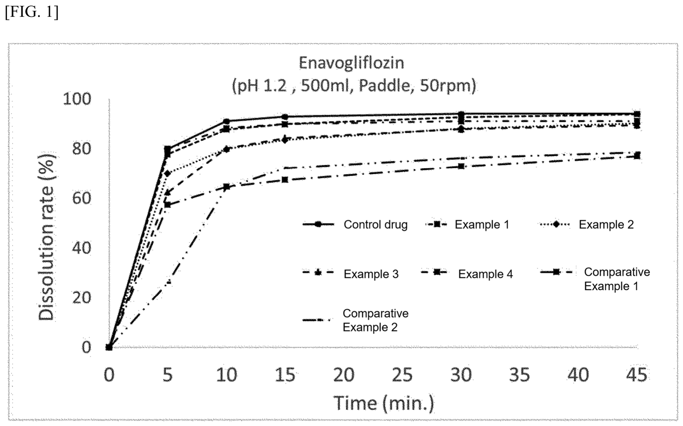

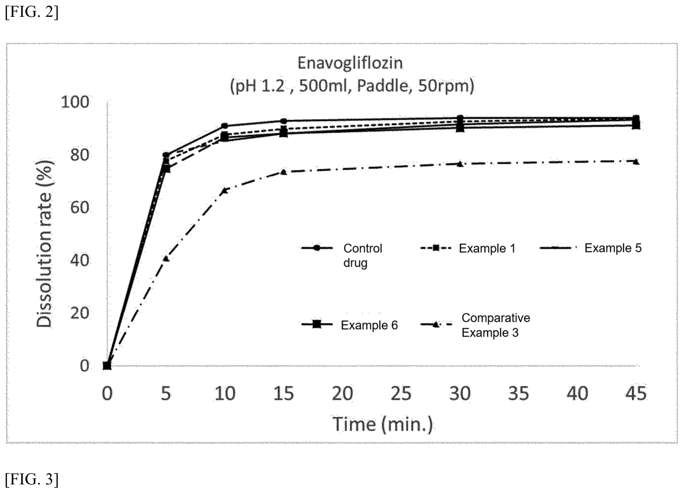

The results of the enavogliflozin dissolution test are shown in Tables 16 to 18 below and FIGS. 1 to 3.

**[Table 16]**

| Dissolution rates of enavogliflozin depending on type of filler | | | | | | |
|---|---|---|---|---|---|---|
| Time (h) | 0 | 5 | 10 | 15 | 30 | 45 |
| Comparator product | 0 | 79.9 | 91 | 92.8 | 94 | 94 |
| Comparative Example 1 | 0 | 57.3 | 64.6 | 67.4 | 72.8 | 76.9 |
| Comparative Example 2 | 0 | 26 | 64 | 72.1 | 76.2 | 78.4 |
| Example 1 | 0 | 77.7 | 87.7 | 89.9 | 92.7 | 93.8 |
| Example 2 | 0 | 70 | 79.8 | 83.4 | 88.1 | 90.1 |
| Example 3 | 0 | 62.3 | 80 | 84.2 | 87.8 | 89.4 |
| Example 4 | 0 | 79.9 | 88.2 | 89.8 | 91 | 91.1 |

**[Table 17]**

| Dissolution rates of enavogliflozin depending on proportion of filler | | | | | | |
|---|---|---|---|---|---|---|
| Time (h) | 0 | 5 | 10 | 15 | 30 | 45 |
| Comparator product | 0 | 79.9 | 91 | 92.8 | 94 | 94 |
| Comparative Example 3 | 0 | 40.8 | 66.6 | 73.7 | 76.7 | 77.7 |
| Example 1 | 0 | 77.7 | 87.7 | 89.9 | 92.7 | 93.8 |
| Example 5 | 0 | 80 | 85.3 | 88 | 91.6 | 93.2 |
| Example 6 | 0 | 74.8 | 86.5 | 88.1 | 90.2 | 91.2 |

**[Table 18]**

| Dissolution rates of enavogliflozin depending on type of disintegrant | | | | | | |
|---|---|---|---|---|---|---|
| Time (h) | 9 | 5 | 10 | 15 | 30 | 45 |
| Comparator product | 0 | 79.9 | 91 | 92.8 | 94 | 94 |
| Comparative Example 4 | 0 | 69.4 | 76.8 | 77.7 | 81 | 83.1 |
| Example 1 | 0 | 77.7 | 87.7 | 89.9 | 92.7 | 93.8 |
| Example 7 | 0 | 81.5 | 86.7 | 87.6 | 90.3 | 91.6 |
| Example 8 | 0 | 73.7 | 83.4 | 83.5 | 85 | 86.8 |
| Example 9 | 0 | 88.1 | 88.3 | 89.4 | 90.5 | 91.7 |
| Example 10 | 0 | 85.7 | 91.2 | 90.6 | 92.7 | 93.8 |

Since enavogliflozin is a component having a Tₘₐₓ of approximately 1 hour, its dissolution in gastric juice is expected to have a significant impact on bioavailability. Accordingly, the dissolution rate at pH 1.2 is considered critical, and Comparative Examples 1 to 4, which do not exhibit a dissolution rate within 10 minutes of 80% or more and a final dissolution rate of 85% or more, are considered undesirable in terms of bioavailability.

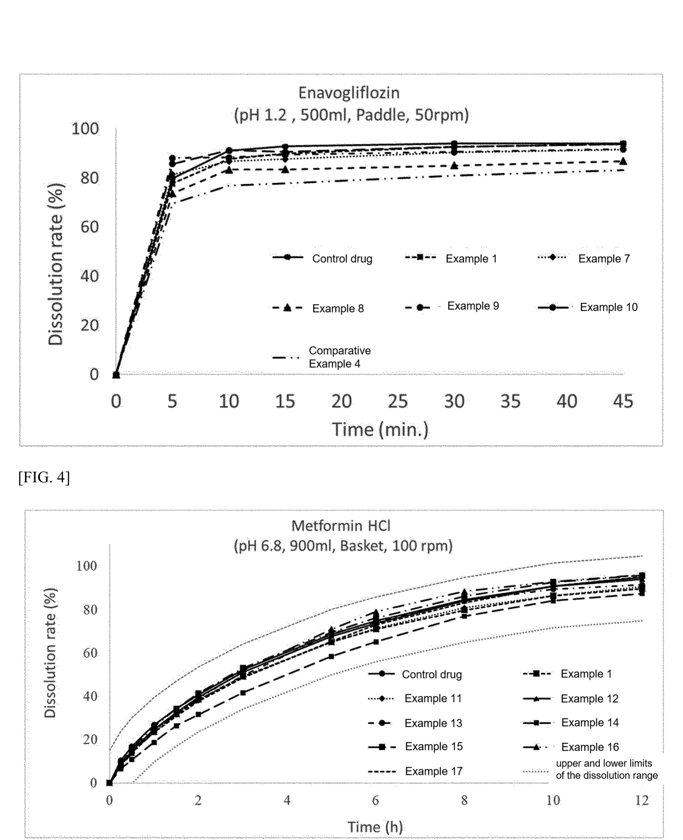

The results of the metformin dissolution test are shown in Table 19 below and FIG. 4.

**[Table 19]**

| Time (h) | 0 | 0.25 | 0.5 | 1 | 1.5 | 2 | 3 | 5 | 6 | 8 | 10 | 12 | f2 |
|---|---|---|---|---|---|---|---|---|---|---|---|---|---|
| Comparator product | 0 | 10.3 | 16.9 | 26.8 | 34.3 | 40.7 | 52.2 | 68.6 | 74.7 | 84.5 | 90.8 | 95.0 | - |
| Example 1 | 0 | 8.8 | 15.0 | 24.6 | 32.0 | 38.5 | 49.1 | 65.0 | 70.9 | 79.8 | 86.5 | 89.7 | 72.66 |
| Example 11 | 0 | 8.2 | 14.7 | 24.4 | 32.0 | 38.2 | 49.1 | 64.9 | 71.2 | 80.8 | 86.5 | 90.5 | 73.67 |
| Example 12 | 0 | 8.3 | 14.9 | 24.7 | 32.6 | 39.6 | 51.0 | 67.7 | 73.5 | 84.0 | 90.7 | 93.9 | 88.56 |
| Example 13 | 0 | 8.0 | 14.1 | 23.7 | 31.6 | 38.3 | 49.5 | 68.9 | 74.2 | 84.1 | 89.4 | 91.4 | 80.16 |
| Example 14 | 0 | 6.5 | 10.8 | 18.7 | 26.5 | 31.7 | 41.6 | 58.4 | 65.0 | 77.0 | 84.0 | 87.4 | 54.34 |
| Example 15 | 0 | 8.9 | 15.7 | 26.0 | 34.3 | 41.4 | 53.0 | 69.4 | 76.1 | 86.2 | 92.6 | 95.8 | 91.13 |
| Example 16 | 0 | 8.0 | 13.8 | 23.5 | 31.9 | 38.9 | 51.8 | 70.9 | 79.0 | 88.4 | 92.8 | 96.0 | 74.90 |
| Example 17 | 0 | 8.5 | 14.2 | 23.4 | 31.0 | 37.7 | 48.5 | 65.3 | 72.9 | 83.1 | 90.8 | 95.1 | 78.29 |

In terms of the metformin dissolution rate, for all Examples, i) the differences in average dissolution rates between the control drug and the test drugs at all points of comparison of dissolution rates, specified in the pharmaceutical equivalence test standards, were approximately 15%, and ii) similarity factors (f2) were 50 or more at all points of comparison of dissolution rates, representing equivalence.

### Experimental Example 5: Clinical trial

A pharmacokinetics test (PK test) was performed on the pharmaceutical composition of Example 1 to evaluate bioequivalence with the control drug.

According to the bioequivalence test based on the pharmaceutical equivalent test standards under the Pharmaceutical Equivalence Act of the Pharmaceutical Affairs-related Law Collection, when both the control drug and the test drug satisfy the 90% confidence interval for the difference in their log-transformed means within the range of log 0.8 to log 1.25, they are regarded equivalent.

A clinical trial was conducted to compare blood drug concentrations after selecting the pharmaceutical composition of Example 1 as a test drug and the combination therapy of Daewoong Pharmaceutical's 'Envlo Tablet' (a single-agent formulation of enavogliflozin), and Merck's 'Glucophage XR, Extended-release tablet, 1,000 mg' (a single-agent formulation of metformin) as a control.

The clinical trial was conducted as a randomized, 2-group and 4-period (pre-meal, post-meal) crossover study, and blood samples were collected at appropriate times after administration. Afterward, pharmacokinetic parameters (AUC and Cₘₐₓ) were calculated and assessed by measuring the concentrations of enavogliflozin and metformin in plasma.

**[Table 20]**

| Pre-meal administration | | | | |
|---|---|---|---|---|
| Enavogliflozin | | | | |
| PK parameters | GMR Ratio | CI_90%_Lower | CI_90%_Upper | Results |
| Cₘₐₓ | 0.9909 | 90.28 | 108.76 | Equivalent |
| AUC | 1.0259 | 93.1373 | 113.00 | Equivalent |

| Metformin | | | | |
|---|---|---|---|---|
| PK parameters | Ratio | CI_90%_Lower | CI_90%_Upper | Results |
| Cₘₐₓ | 0.9979 | 83.81 | 118.81 | Equivalent |
| AUC | 0.9576 | 81.41 | 112.64 | Equivalent |

**[Table 21]**

| Post-meal administration | | | | |
|---|---|---|---|---|
| Enavogliflozin | | | | |
| PK parameters | Ratio | CI_90%_Lower | CI_90%_Upper | Results |
| Cₘₐₓ | 0.8759 | 0.82 | 0.93 | Equivalent |
| AUC | 1.0347 | 0.98 | 1.09 | Equivalent |

| Metformin | | | | |
|---|---|---|---|---|
| PK parameters | Ratio | CI_90%_Lower | CI_90%_Upper | Results |
| Cₘₐₓ | 1.0147 | 0.98 | 1.05 | Equivalent |
| AUC | 1.0038 | 0.97 | 1.04 | Equivalent |

The test demonstrated that the pharmaceutical composition of Example 1 exhibits bioequivalence with the combination therapy with the control drug both before and after eating.

## Claims

1. A pharmaceutical composition in a single dosage form comprising:
a compartment including metformin or a pharmaceutically acceptable salt thereof and a compartment including enavogliflozin or a pharmaceutically acceptable salt thereof, wherein the compartments are formulated in a separated form from each other,
wherein the enavogliflozin compartment is included at 10 to 20 parts by weight with respect to 100 parts by weight of the entire pharmaceutical composition, and
wherein enavogliflozin or a pharmaceutically acceptable salt thereof in the enavogliflozin compartment is included at less than 0.3 parts by weight with respect to a total of 100 parts by weight of the enavogliflozin compartment.

2. The pharmaceutical composition of claim 1, wherein the enavogliflozin compartment comprises enavogliflozin or a pharmaceutically acceptable salt thereof, an excipient, a disintegrant, and a glidant, and the metformin compartment comprises metformin or a pharmaceutically acceptable salt thereof, a binder, a sustained-release agent, and a glidant.

3. The pharmaceutical composition of claim 1, wherein the enavogliflozin compartment comprises an excipient selected from the group consisting of lactose monohydrate; mannitol; a mixture of microcrystalline cellulose and lactose monohydrate; and a mixture of microcrystalline cellulose, mannitol and pregelatinized starch, and the excipient is included at 80 to 85 parts by weight with respect to a total of 100 parts by weight of the enavogliflozin compartment.

4. The pharmaceutical composition of claim 1, wherein when the enavogliflozin compartment comprises lactose monohydrate as an excipient, the lactose monohydrate is included at 40 to 100 parts by weight with respect to a total of 100 parts by weight of all excipients in the enavogliflozin compartment.

5. The pharmaceutical composition of claim 1, wherein when the enavogliflozin compartment comprises mannitol as an excipient, the mannitol is included at 20 to 100 parts by weight with respect to a total of 100 parts by weight of all excipients in the enavogliflozin compartment.

6. The pharmaceutical composition of claim 1, wherein, when the enavogliflozin compartment comprises microcrystalline cellulose as an excipient, the microcrystalline cellulose is included at less than 65 parts by weight with respect to a total of 100 parts by weight of all excipients in the enavogliflozin compartment.

7. The pharmaceutical composition of claim 1, wherein, when the enavogliflozin compartment comprises pregelatinized starch as an excipient, the pregelatinized starch is included at 5 to 40 parts by weight with respect to a total of 100 parts by weight of all excipients in the enavogliflozin compartment.

8. The pharmaceutical composition of claim 1, wherein, when the enavogliflozin compartment comprises microcrystalline cellulose, mannitol, and pregelatinized starch as excipients, the mannitol is included at 20 to 65 parts by weight with respect to a total of 100 parts by weight of all excipients in the enavogliflozin compartment.

9. The pharmaceutical composition of claim 1, wherein, when the enavogliflozin compartment comprises microcrystalline cellulose, mannitol, and pregelatinized starch as excipients, the microcrystalline cellulose, mannitol and pregelatinized starch are included at a weight ratio of 1:0.5 to 2:0.15 to 1.5 in the enavogliflozin compartment.

10. The pharmaceutical composition of claim 1, wherein the enavogliflozin compartment comprises a disintegrant selected from the group consisting of croscarmellose sodium, sodium starch glycolate, crospovidone, and low-substituted hydroxypropyl cellulose, and the disintegrant is included at 5 to 20 parts by weight with respect to a total of 100 parts by weight of the enavogliflozin compartment.

11. The pharmaceutical composition of claim 1, wherein the enavogliflozin compartment comprises one or more of light anhydrous silicic acid and talc as a glidant, and the glidant is included at 1.5 to 3 parts by weight with respect to a total of 100 parts by weight of the enavogliflozin compartment.

12. The pharmaceutical composition of claim 1, wherein the metformin compartment comprises carboxymethylcellulose sodium, povidone, or a mixture thereof as a binder, and the binder is included at 2 to 5 parts by weight with respect to a total of 100 parts by weight of the metformin compartment.

13. The pharmaceutical composition of claim 1, wherein the metformin compartment comprises one or more of hydroxypropyl methylcellulose and oxidized polyethylene as a sustained-release agent, and the sustained-release agent is included at 15 to 40 parts by weight with respect to a total of 100 parts by weight of the metformin compartment.

14. The pharmaceutical composition of claim 1, wherein the metformin compartment comprises magnesium stearate as a glidant at 0.5 to 1 part by weight with respect to a total of 100 parts by weight of the metformin compartment.

15. The pharmaceutical composition of claim 1, wherein the dissolution rate of the enavogliflozin or a pharmaceutically acceptable salt thereof in a pH 1.2 dissolution solution after 10 minutes is 80% or more with respect to the total content of the enavogliflozin or a pharmaceutically acceptable salt thereof.

16. The pharmaceutical composition of claim 1, wherein the dissolution rate of the enavogliflozin or a pharmaceutically acceptable salt thereof in a pH 1.2 dissolution solution after 45 minutes is 85% or more with respect to the total content of the enavogliflozin or a pharmaceutically acceptable salt thereof.

17. The pharmaceutical composition of claim 1, wherein the dissolution rate of the metformin or a pharmaceutically acceptable salt thereof in a pH 6.8 dissolution solution after 1 hour is 20% or more with respect to the total content of the metformin or a pharmaceutically acceptable salt thereof.

18. The pharmaceutical composition of claim 1, wherein the dissolution rate of the metformin or a pharmaceutically acceptable salt thereof in a pH 6.8 dissolution solution after 3 hours is 45% or more with respect to the total content of the metformin or a pharmaceutically acceptable salt thereof.

19. The pharmaceutical composition of claim 1, wherein the dissolution rate of the metformin or a pharmaceutically acceptable salt thereof in a pH 6.8 dissolution solution after 12 hours is 85% or more with respect to the total content of the metformin or a pharmaceutically acceptable salt thereof.

20. The pharmaceutical composition of claim 1, wherein the enavogliflozin compartment comprises a granulate in which pre-mixed granules including enavogliflozin or a pharmaceutically acceptable salt thereof are mixed with a post-mixed part.

21. The pharmaceutical composition of claim 20, wherein the pre-mixed granules comprise enavogliflozin or a pharmaceutically acceptable salt thereof, an excipient, and a glidant.

22. The pharmaceutical composition of claim 20, wherein the post-mixed part comprises an excipient, a disintegrant, and a glidant.

23. The pharmaceutical composition of claim 20, wherein each of the pre-mixed granules and the post-mixed part comprise one or more excipients.

24. The pharmaceutical composition of claim 20, wherein the weight ratio of the excipient in the pre-mixed granules and the excipient in the post-mixed part ranges from 1:1 to 1:4.

25. The pharmaceutical composition of claim 20, wherein the granulate is a dry granulate.

26. The pharmaceutical composition of claim 1, wherein the metformin compartment comprises a granulate in which pre-mixed granules including metformin or a pharmaceutically acceptable salt thereof are mixed with a post-mixed part.

27. The pharmaceutical composition of claim 26, wherein the granulate is a wet granulate.

28. The pharmaceutical composition of claim 1, wherein the pharmaceutical composition is in the form of a tablet.

29. The pharmaceutical composition of claim 1, wherein the pharmaceutical composition is in the form of a bilayer tablet.

30. The pharmaceutical composition of claim 1, wherein the pharmaceutical composition comprises enavogliflozin at a dose of 0.1 to 0.5 mg.

31. The pharmaceutical composition of claim 1, wherein the pharmaceutical composition comprises metformin at a dose of 500 to 1,000 mg.
